(19)

Europäisches
Patentamt

European
Patent Office

Office européen
des brevets

(11) **EP 3 357 414 A1**

(12) **EUROPEAN PATENT APPLICATION**

(43) Date of publication:
**08.08.2018 Bulletin 2018/32**

(51) Int Cl.:
*A61B 5/00* (2006.01)   *A61B 5/02* (2006.01)
*A61B 5/20* (2006.01)   *C09D 11/52* (2014.01)

(21) Application number: **17382045.7**

(22) Date of filing: **01.02.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Universitat Rovira I Virgili
43003 Tarragona (ES)**

(72) Inventors:
• **ANDRADE, Fancisco Javier
43007 Tarragona (ES)**

• **FERRÉ ALBIOL, Jordi
43007 Tarragona (ES)**
• **BLANKING, Pär Robert Erik William
43007 Tarragona (ES)**
• **GUINOVART PAVÓN, Tomás de Aquino
43007 Tarragona (ES)**
• **NOVELLS RECASENS, Marta
43007 Tarragona (ES)**
• **PARRILLA PONS, Marc
43007 Tarragona (ES)**

(74) Representative: **Hoffmann Eitle
Hoffmann Eitle S.L.U.
Paseo de la Castellana 140, 3a planta
Edificio LIMA
28046 Madrid (ES)**

(54) **BIOSENSOR FOR DETECTING BIOLOGICAL FLUID**

(57)     Disclosed is a non-therapeutic use of a sensor for detecting biological fluids *in vitro,* the sensor comprising a substrate coated with conductive ink, the substrate being inert relative to the conductive ink, the sensor comprising means for measuring conductivity or resistivity of the conductive ink, wherein the conductive ink comprises a carbon substrate and a surfactant. The sensor may also comprise a polymer such as a gum. Also disclosed are methods of detecting biological fluids and uses of the sensor.

Fig. 12

Area < $0,2cm^2$

R

## Description

### Field of the Invention

The present invention relates to a sensor for detecting biological fluids. In particular, the invention relates to a sensor made with a coating with a conductive ink film comprising a carbon substrate. A surfactant and/or a polymer may also be present in the ink film. The present invention also relates to non-therapeutic uses of the sensor and non-therapeutic methods of detecting a biological fluid *in vitro*.

### Background of the Invention

Sensors for detecting moisture -particularly moisture in air in the form of water vapour- are known in the art. For example, US Patent Application, US 2014/0182372 A1 describes a water vapour sensor which may be used to detect humidity. The water vapour sensor comprises a substrate (made of plastic, ceramic glass, silicon, or any other material that is inert to the film); a film of carbon nanotubes impregnated with surfactant; and electrical conductors. The sensor is contained in a housing that is said to mechanically protect it from damage and although the sensor may be used to detect humidity, water droplets should not be formed on the sensor as they may erode surfactant from the film.

Mäder et al. (J. Mater. Chem. A, 2014, 2, 5541-55447) disclose the production of multi-walled carbon nanotube-cellulose fibres and their use as water sensors. When immersed in water, the cellulose fibres swell and the conductive carbon nanotube networks disconnect. When the fibres are dried the cellulose fibres shrink and the disconnected nanotubes approach each other and can form an electrical conducting network. The wetting and drying of the cellulose fibres can be used to form an electrochemical switch.

Xing et al. (ACS Appl. Mater. Interfaces, 2015, 7, 26195-26205) disclose a carbon nanotube-based strain gauge to monitor bodily motion and also to monitor humidity changes with high sensitivity and a fast resistance response capable of monitoring human breathing. They found that as humidity increases, resistance decreased exponentially. Furthermore, they found that by increasing the carbon nanotube concentration the resistance decreases, and at 7% CNT content, the resistance remains unchanged for all humidity values.

Despite the advances in moisture and fluid detectors, there still exists a need in the art for a sensor for detecting biological fluids having improved properties, such as increased sensitivity, compared to the sensors known in the art.

### Summary of the Invention

The inventors of the present disclosure have surprisingly found that a sensor comprising a conductive ink coating a substrate and means for measuring the electrical resistance of the ink, can be used to detect the presence of biological fluids and -under certain conditions also quantify their amount- with improved properties, such as increased sensitivity. In this sense, a conductive ink, understood as any type of dispersion of carbon substrates in a water-based solvent by using any form of dispersing chemical agent, coated on an inert substrate to create a film or a layer, provides for an improved sensor capable of detecting and measuring fluids such as biological fluids in very low amounts.

The sensor may be used to detect any type of biological fluid, such as blood, serum, sweat, saliva, urine, or any other water-based body secretion. The sensor may be incorporated into a disposable or non-disposable article and used to detect biological fluids *in vitro* and *in vivo*. For example, the sensor may be incorporated into a wearable device. Non-limiting examples of wearable devices include plasters, bandages, adhesive patches, diapers, clothing or footwear.

### Brief description of the drawings

In all figures, sensors have been built using as a substrate a commercial filter paper. The conductive ink has been applied by painting using a conventional paintbrush. The sensors are prepared by applying a layer of ink and air-drying. Once the layer is dried, the electrical resistance of the paper is measured using a conventional digital multimeter. This process of painting and drying is repeated until a target value of electrical resistance is obtained. This target value is usually set in the order of 30 $\Omega$/cm, but can be adjusted depending on the number of layers applied. Once the conductive paper is ready, rectangular stripes of a given dimension (depending on the experiment) are cut. These sensing strips were placed on a suitable holder to perform two point electrical resistance measurements the two distant ends of the strip. Small variations in the initial resistance between strips may be observed due to the manual manufacturing process employed.

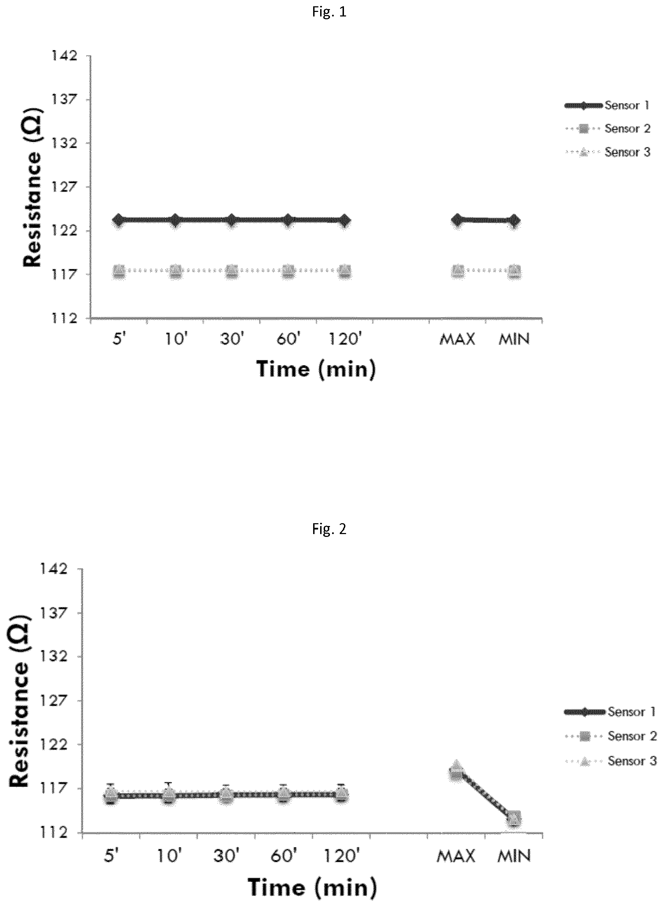

Figure 1: short, medium and long-term stability of three biosensors following the Gold Standard Method.
This figure aims to illustrate the stability of the measurement of the resistance when using a sensor that is not exposed to water.

The conductive ink used to make the sensors contains 3 g of single-walled carbon nanotubes per 100 mL of solution. 10 % sodium dodecylbenzene sulphonate (SDBS) was used as a surfactant. The dimensions of the sensing stripes were 5 cm x 1 cm.

The resistance of each sensor individually was monitored as a function of the time. Each point in the graph corresponds to an average of the value within the corresponding time interval. Stability shows a standard deviation (STDV) of $0.00\ \Omega \pm (0.00)$, $0.00\ \Omega \pm (0.00)$, $0.01\ \Omega \pm (0.00)$, $0.01\ \Omega \pm (0.00)$ and $0.02\ \Omega \pm (0.01)$ at 5, 10, 30, 60 and 120 min for each one of three sensors tested respectively. None of the sensors tested showed peak to peak variabilities larger than $0.1\ \Omega$ after 120 min of continuous measurement.

These experiments allow concluding that variations in the resistance down to $0.1\ \Omega$ can be easily detected.

Figure 2: short, medium and long term stability of three biosensors using a wireless device
This figure aims to illustrate the possibility of using a compact and portable wireless device to monitor the resistance of the sensor.

The sensors are similar to those described in Figure 1, with an initial resistance of $120\ \Omega$. The sensors were connected to the wireless device and and the resistance of sensors that have not been exposed to any water was monitored as function of the time. Each point in the graph corresponds to an average of the value within the corresponding time interval. Standard deviations of $0.80\ \Omega \pm (0.04)$, $0.82\ \Omega \pm (0.09)$, $0.73\ \Omega \pm (0.03)$, $0.72\ \Omega \pm (0.01)$ and $0.71\ \Omega \pm (0.00)$ at 5, 10, 30, 60 and 120 min for three sensors tested respectively. Peak to peak variability was set at $5.6\ \Omega \pm (0.6)$ at 120 min for the three sensors.

This figure confirms that the resistance of the sensor can be wirelessly monitored using a compact device. In this case minimum detectable levels increase to approximately $2\ \Omega$. By comparing this result with those in Figure 1 it is clear that this is not an inherent limitation of the sensor, but of the electronic device used. Further improvements in the electronics may yield better results.

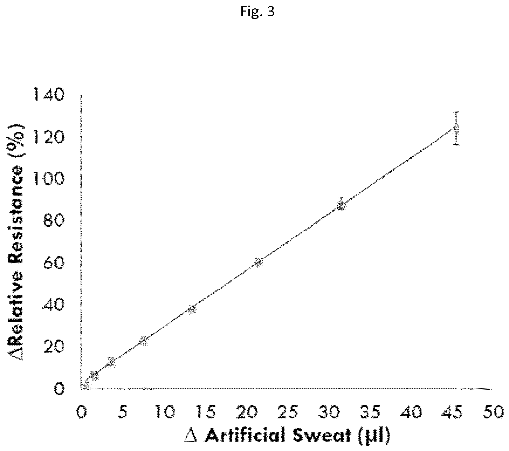

Figure 3: Relative change in resistance (%) when exposed to sweat and/or other biological fluid.
The sensors illustrated in this figure were prepared and the measurements were performed under the same conditions described in Figure 1.

In this case, discrete volumes of water and/or a saline solution with different amount of dissolved salts (up to an equivalent of 0.1 molar sodium chloride) were added in the center of the sensing strip while the resistance was monitored as a function of the time. A key observation regarding this invention is that the electrical resistance increases with the addition of solution. Therefore, one can calculate the relative change in electrical resistance ($\Delta$R%) as a function of the amount of solution added, as shown in the plot of Figure 3. No difference in the response was found between the use of water or solutions with different concentrations of dissolved salts.

This plot shows that $\Delta$R% changes linearly with the total amount of liquid added, and that this can be used to estimate the total amount of water (or saline solution) present in the sensing strip. The slope of the plot of $\Delta$R% vs. amount of liquid added is referred as the sensitivity of the sensor.

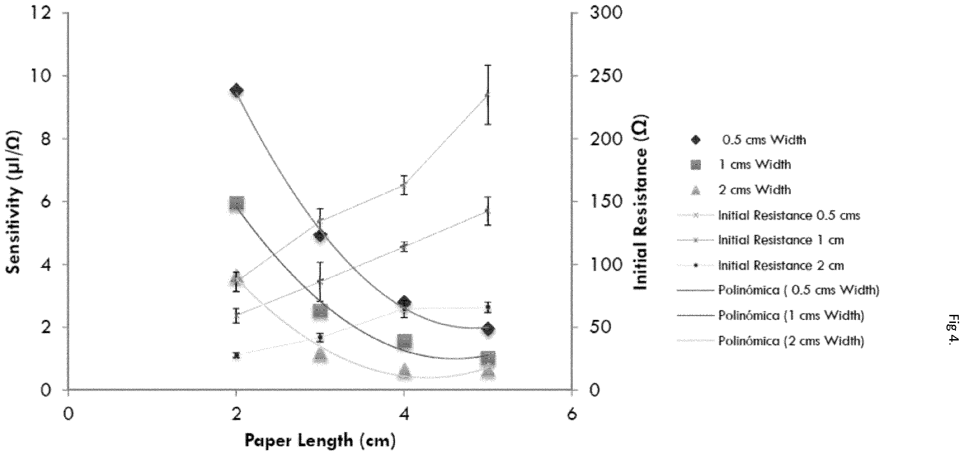

Figure 4: Effect of the geometry of the sensor on the sensitivity.
The sensors illustrated in this figure were prepared and the measurements were performed under the same conditions described in Figure 1. In this case, sensing strips of different lengths and widths were used, in order to evaluate the effect of the sensor's geometry on the sensitivity. First, this plot shows that the sensing strip initially behaves as expected from an electronic conductor: the resistance increases with the length (for a given width), and decreases with the width (for a given length). Second, it shows that the sensitivity for the sensing strip decreases with the length and with the width. In other words, shorter and thinner sensing strips show higher sensitivity. This information is useful when designing the sensing strips for targeted applications.

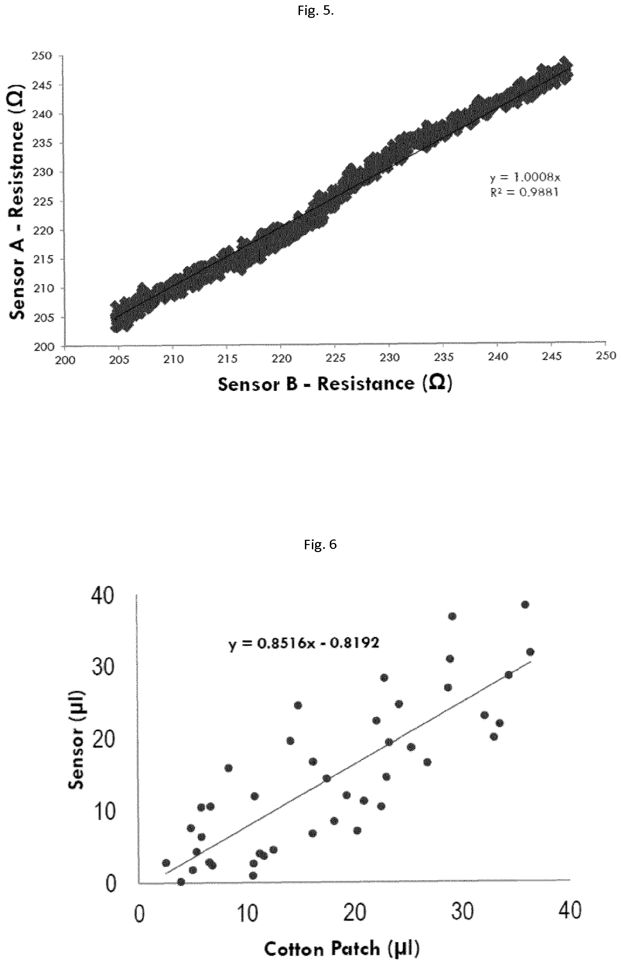

Figure 5: Reproducibility of response between sensors.
The sensors illustrated in this figure were prepared as stated in Figure 1. In this case, two different sensors are independently calibrated as described in Figure 3. Thereafter, the measured change on the electrical resistance for one of the sensors is plotted against the change on the electrical resistance observed for the other. The plot shows that both sensors show comparable response, demonstrating the good reproducibility between two different sensors.

Figure 6: validation of the sensor response against standard methods.
The sensors illustrated in this figure were prepared as described in Figure 1. In this case, the sensor was used as a wearable patch, on the skin surface, to monitor the sweat of a person.

To do this, the sensing strips were mounted on a holder, as described below, and adhered onto the skin of a subject on selected anatomical regions. Several sensors were located on four different anatomical regions (forearm, upper back, lower back and chest). Close to each sensor, sweat adsorbing cotton pads with a normalized surface area were located following the standard protocols to monitor the sweat loss. After a given time, the reading of the sensor was used to calculate the amount of sweat loss. In parallel, the cotton pad was removed and weighted to monitor the actual amount of sweat collected. This is the gold standard method and was used to validate the reading of the sensor. The figure compares the values calculated by the sensors with those obtained for the conventional cotton patch sensor. It should be stressed that the methodology of the cotton patch is prone to significant error, particularly at this low level. In any case, the good correlation between the data validates the use of the sensor to monitor sweat loss.

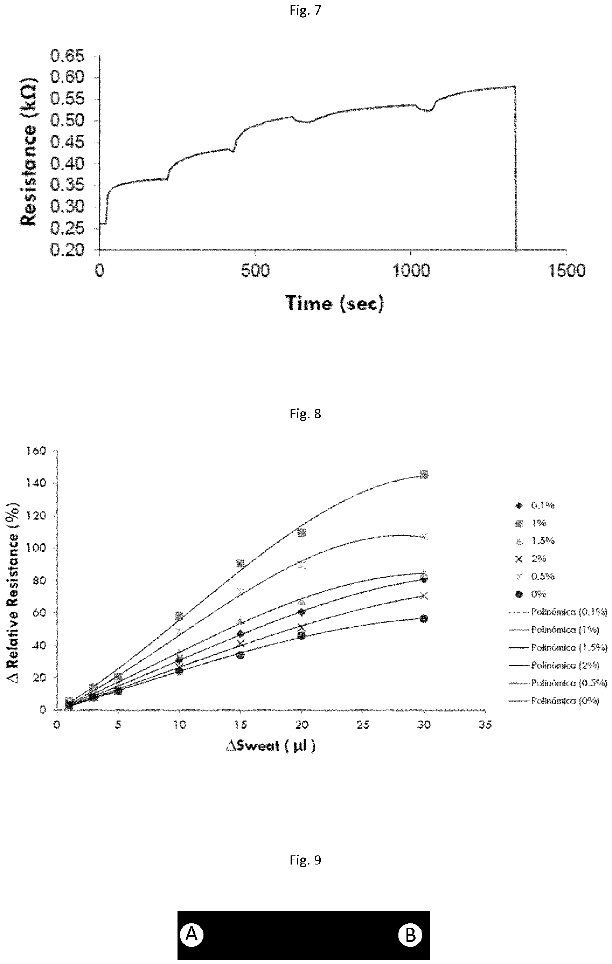

Figure 7: Results of the smart diaper in adults.
The sensors illustrated in this figure were prepared as described in Figure 1. In this case, the sensor was placed within the absorbent area of a commercial diaper Thereafter, the relative change on the electrical resistance of the sensor was monitored as a function of the time while water was added to the diaper showing that the sensor can be used to monitor the amount of liquid present in the diaper. Similar experiments conducted with artificial and real urine showed comparable results.

Figure 8: Effect of different concentrations of Gum Arabic on the sensitivity of the sensor
The sensors illustrated in this figure were prepared as described in Figure 1. In this case, however, the ink was preparing by adding also a given amount of Gum Arabic . The graph demonstrates that the relative change on resistance can be modified by changing the fraction of Gum arabic. In the present experiments optimum sensitivities is achieved with concentrations between 0.5 and 1% of Gum Arabic.

Figure 9. This figure illustrates the points where the resistance is measured in the sensor: at the two extremes of the conductive paper, between points A and B.

Figure 10. Short and medium term stability of the sensors (%).
The sensors illustrated in this figure were prepared as described in Figure 1. The results show the sensitivity obtained for a batch of sensors used during 8 consecutive weeks. This plot shows that the sensitivity of the sensor remains constant as a function of the added sweat during a period time of 8 weeks after production.

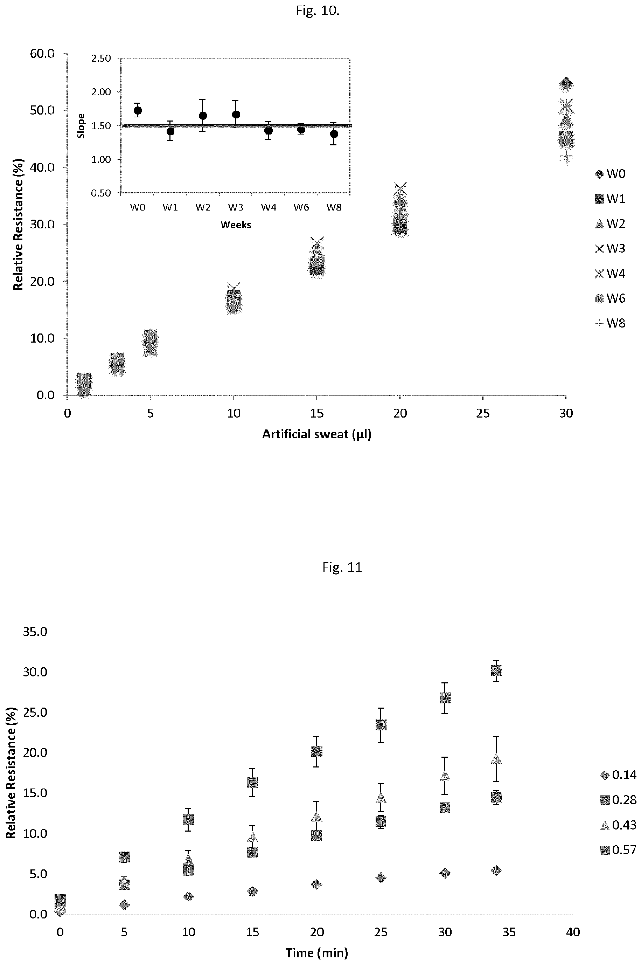

Figure 11. Evaluation of the rate of liquid addtion on the sensor's response.
The sensors illustrated in this figure were prepared as described in Figure 1 with the addition of 0.01 mg/mL of Arabic gum. Experiments were conducted to determine whether the rate of addition of liquid has any influence on the response of the sensor. Liquid was added to the sensor at flow rates ranging from 0.14 to 0.57 $\mu$L/min, which can be considered close between low to high physiological ranges (considering the area of the sensor). This experiment provides important information taking into account that sweat rate is non uniform for a given person and across different people. The plot does not show significant differences in the response, stressing the idea that the rate of addition of liquid does not have an influence on the sensor's response.

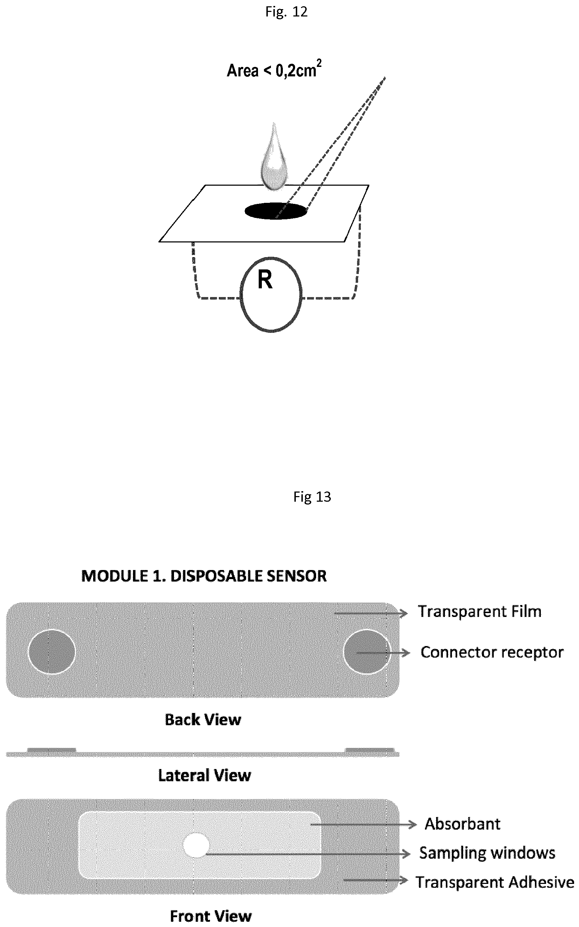

Figure 12. Biological fluid is added and the resistance is monitored as function of time.

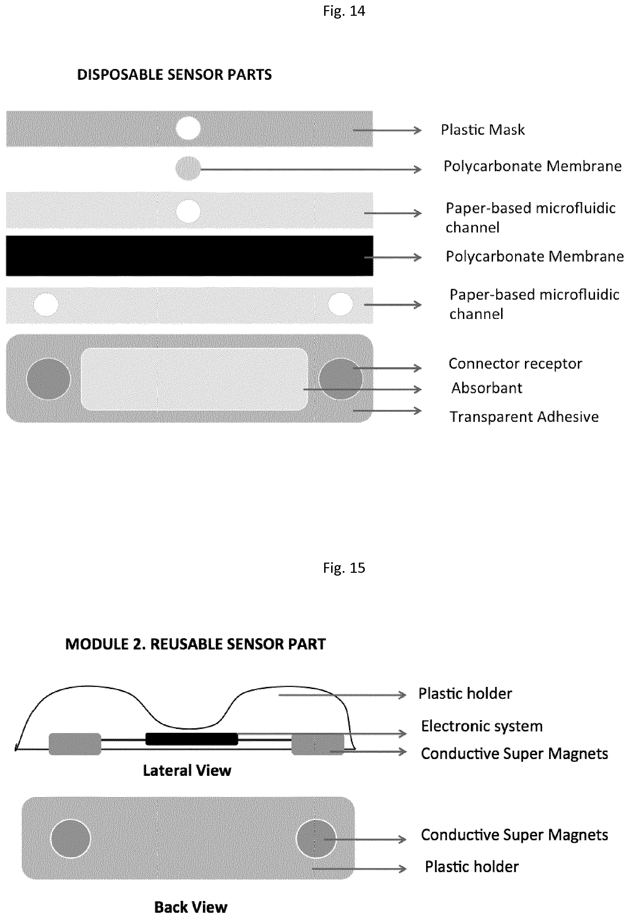

Figures 13 and 14. Sensor's disposable modules.

Figure 15. Sensor's non-disposable module.

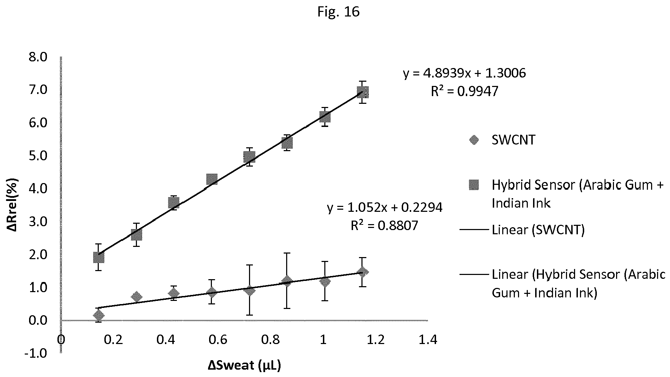

Figure 16. Relative Resistance (%) represented as a function of the sweat amount in response of two different ink compositions.

Detailed description

The sensor

The present invention is based on the changes produced by the presence of water-based solutions on the measurement of the electrical resistance of a novel sensing device. In particular, a sensor (from hereinafter the sensor/s of the present invention) comprising a substrate that is coated with a conductive ink, the substrate being inert relative to the conductive ink, the sensor comprising means for measuring conductivity or resistivity of the conductive ink, wherein the conductive ink comprises a carbon substrate and optionally other components, such as one or more surfactants. Additionally, some polymers (such as, but not restricted to) gum Arabic can be used to modify the sensitivity of the sensor.
It is noted that the conductive ink can be any type of dispersion of carbon substrates in a water-based solvent by using some form of dispersing chemical agent.

The carbon substrate in the conductive ink may be selected from the group comprising carbon nanostructured materials, amorphous carbon, graphite, and any mixtures thereof. The carbon nanostructured materials may be carbon nanotubes. The carbon nanotubes may be single walled carbon nanotubes (SWCNT), multiwalled carbon nanotubes (MWCNT), or any mixtures thereof. Preferably the carbon substrate is graphite or carbon nanotubes.

It is noted that the carbon substrates useful in the present invention are not soluble in water, such as carbon nanotubes. Thus, when in contact with water, they form agglomerates or bundles that do not allow making a homogenous dispersion and therefore are detrimental for the generation of a conductive material through a printing process. To disperse them properly, a chemical agent that can disaggregate these bundles and keep them dispersed is necessary. Surfactants, in this sense, should be used, because they can interact with the carbon-based materials through the non-polar end and with the solvent (through the polar or charged end) simultaneously. Examples of surfactants that can be used in the present invention without affecting the properties oft he sensor may be selected from (but not limited to) the group consisting of cationic surfactants, anionic surfactants and neutral surfactants, such as salts of dodecylbenzensulphonate (SDBS), dodecylsulphonate (SDS), polystyrene sulphonate (PSS), triton, etc. Some other materials, such as chitosan or Nafion(R), can be also used to disperse carbon substrates. Dispersion may be assisted by means of heating and or applicaiton of ultrasonic treatment, as it is well described in the literature. Once the carbon materials aredispersed in the system forming the ink, they can be applied directly onto the substrate by a direct printing or dyeing process.

The conductive ink may further comprise a polymer selected from the group comprising polysaccharides or gums. Preferably the conductive ink comprises a gum selected from the group comprising natural gum, or Gum Arabic. Preferably the gum is Gum Arabic.

The conductive ink may further comprise an ink, such as Indian ink.

The substrate may be any sort of material that displays two main properties:

1) Produces strong absorption of the carbon substrate (such as the carbon nanotubes or the graphite); and

2) Suffers changes when exposed to water -such as swelling- that will alter the electrical properties of the material.

The substrate is preferably selected from the group comprising cellulose-based derivates such as (but no limited to) paper or cotton, hydrophilic polymeric materials, such as polyacrylates and methacrylate, gum, and combinations thereof. Substrates and combinations thereof will be chosen depending on the use of the sensor. It is, however noted that cellulose-based materials are ideally suited fort he present invention since they display both properties. Interestingly, due to the possibility to produce inks with carbon nanotubes, the current device does not require the manufacturing of a special composites. In the sensors of the present invention, the substrate can be in the form of a sheet, where the carbon substrate ink can be painted with any type of direct printing method (roll to roll, paintbrush, aerograph, etc.) Once the water-based ink is dried, the system can be used as a sensor.

In addition to the substrate coated with a conductive ink, the sensor further comprises means for measuring the electrical resistance of the conductive ink-substrate system. In order to simplify the measurements, the substrate coated with the conductive ink is cut in the form of rectangular strips and two conductive pads are placed at the most distant edges of the rectangle. These conductive pads are glued to the conductive strip using any conductive glue available in the market. This step, is aimed to facilitate a good electrical connection between the conductive strip and the measuring device. In order to measure the electrical resistance of the conductive strip the two terminals of a suitable measuring device are connected to the metallic pads. To this end, the system comprises a module with a connector holder and the electronic system to measure the electrical resistance. Additionally, this module may include components that allows the processing and communication to other electronic components either with cables or through some wireless communication protocol.

It is important to stress that -unlike many other methods- the proposed approach measures the electrical resistance, i.e., it works under direct current and does not require the use of alternate current during the measurement. This simplifies the measuring approach and the instrumentation required. The procedure described above makes use of the 2-point measuring approach, which provides enough accuracy for the applications of this work. Alternatively, if more metal pads are added along the axis of the sensing strip, 4 point measurements can be also performed to obtain a more accurate measurement of the electrical resistance.

In a preferred embodiment of the first aspect of the invention, the invention provides a plug and play system allowing the connection between the substrate coated with a conductive ink and the module comprising the connector holder and the electronic system that allows the signal processing and communication.

In order to to build this plug an play system the conductive pads that are used as electrical connectors are made using any kind of ferromagnetic material -such as iron, steel, galvanized metal, ferromagnetic composites materials, etc.,. Then, the two ends of the measuring device are made with magnets strong enough to simply bind by magnetic force to the conductive pads. In this way, the measuring device can be connected or disconnected simply approaching the connector or by pulling it out, respectively.

Therefore, a first aspect of the invention refers to a sensor (from hereinafter the sensor/s of the present invention) as defined above, comprising a substrate coated with a conductive ink, the substrate being inert relative to the conductive ink, the sensor comprising means for measuring conductivity or resistivity of the conductive ink, wherein the conductive ink comprises a carbon substrate and optionally one or more surfactants. Preferably, the means for measuring conductivity or resistivity of the conductive ink are provided by a module comprising the connector holder and the electronic system that allows the signal processing and communication as described above. More preferably, the connection between the substrate coated with a conductive ink and the module comprising the connector holder and the electronic system that allows the signal processing and communication is performed by a plug and play system.

When dealing with the foundations of electrical and electronic circuits, there are three fundamental magnitudes, namely: voltage (V), current (I) and resistance (R). In metallic conductors -such as a carbon substrate as carbon nanotubes- these magnitudes are related by the Ohm's law:

$$V = I * R$$

Which means that by measuring two of them, the third one is calculated. Many of the commercial instruments used to measure electrical resistance make use of this relationship. In any case, the measurement of the electrical resistance is one of the most basic and fundamental activities for any person dealing with electrical or electronic circuits. Thus, any person with a minimum knowledge on the fields of electrical circuits, electricity or electronics will find obvious the measurement of the electrical resistance.

It is important to point out that the sensor of the present invention works with direct current (DC), so no need to use pulsed, radiofrequency or any alternate current schemes are needed. In this sense, it is noted that in the literature other devices make use of these power schemes, such as radiofrequency making the design of these systems more complex, because they have to use interdigitated electrodes, and what they measure is the impedance of the system. In the design proposed for the sensor of the present invention, the use of direct current allows for the simplification of the measurement system in the following ways:

1) The design of the electrode is simplified (just a conductive strip)
2) The measuring device is simpler (no need to read at a given frequency)
3) The susceptibility to noise and external radiation is minimized, which is particularly important when measuring with wireless devices.

As mentioned before, the measuring device requires (at least) only 2 terminals (A and B), each one connected at each extreme of the sensor as illustrated in figure 9. Alternatively, if a four point measurement is required, two additional terminals along the axis can be added, and standard procedures well described in the literature can be followed. In said figure, one terminal of the measuring device will be located at point A, and the second terminal at point B. Thus, the electrical resistance across the sensor will be measured.

Regarding the measuring instrument, virtually any device with ability to measure electrical resistance with a precision of at least 1 ohm will be useful with the current sensors. Thus, commercial voltmeters that can be acquired in any street shop could potentially be used.

It is noted that the main use of the sensor/s of the present invention, as illustrated through-out the present specification below, is for detecting biological fluids. The biological fluids detected by the sensor include fluids selected from the group comprising sweat, urine, blood and saliva. The biological fluids detected by a sensor of the present invention may be produced by a mammal such as a human, a horse, a cow a dog, a camel, a donkey or a pig. In a preferred embodiment the mammal is a human.

The sensor according to the present disclosure may be disposable or non-disposable. The sensor may be used and dried according to any know method. Examples of such methods include heating, drying in air and blow drying. The sensor may be used multiple times. Preferably the sensor is used once before being discarded.

The sensor according to the present disclosure is preferably in the form of a wearable device included in or present in, for example, plasters, bandages, adhesive patches, tattoos, diapers, watches, wristbands, caps, fibres, yarns, compressive garments, clothing or footwear.

Manufacturing methods of the sensor.

The manufacturing method of the sensor present the following steps:

I) The first step refers to the development of a conductive ink. A carbon-based ink (Cink) was elaborated by adding a carbon-based material to a surfactant solution. In an example (but not limited to it) a carbon nanotubes ink (CNT ink) was made by adding single walled carbon nanotubes (SWCNTs) to a 10 mg/mL sodium dodecylbenzenesulfonate (SDBS) aqueous solution. A optimized concentration of 3 mg/mL of SWCNT was used since it gives optimum ink stability. Furthermore SWCNT were successfully dispersed using a commercial tip sonicator between 45 min to 120 min. Typical conditions for sonication are 100 W, frequency of 24 KHz, 60 % of amplitude and a cycle of 0.5 s. In addition a 0.01 mg/mL of Arabic gum and/or also 0.01 mg/mL of Indian ink could be added and dispersed with the sonicator for 10 min once the CNT-ink was heated for 5 min around 80°C. Under these conditions and as shown in figure 10, the conductive ink can be used for making sensors for at least 3 months with good analytical performance. II) The second step refers to the process of characterization of a substrate material from the group comprising paper, cotton, gum, carbon fibres and combination thereof. Ideally, cellulose-based materials are suited due to the capabilities to; a) produce strong absorption of the carbon substrate; and, b) suffers changes when exposed to water - such as swelling - that will alter the electrical properties of the material. In that sense, a conventional and commercial filter paper is painted with the conductive ink by both sides using any type of direct printed method. The ink is readily absorbed by the paper and, after the solvent evaporates (usually few minutes), the papers is thoroughly rinsed with water. Some bubbles are observed during the rinsing process showing that, to some extent, the excess of surfactant is being washed out. After the rinsing, the paper is dried at room temperature or, to speed up the process, it can be placed in an oven at 60°C for about 5 minutes. At this point the resistance is checked for optimization purposes. This process of painting, drying, washing out the surfactant, and drying again is considered one cycle. High percolation of the deposited CNTs, Arabic gum and Indian Ink was obtained when more painting cycles were performed. However, a percolation threshold or saturation value of electrical properties was reached with extensively over-lapping SWCNTs networks after a high number of painting repetitions. After 4 painting cycles at both paper sides, the conductive paper reached a steady-state resistance value, usually around 300 $\Omega$/cm.

III) The third step refers to the assemble of the wearable sensor and the integration into commercial available products such as adhesive patches, plasters, bandages. As an example, a wearable patch is firstly made by cutting the conductive paper-based into customized strips that as can be seen in figure 4 present different analytical performance based on its width and length, respectively. Taking into account a standard, not limiting, strip size of 1 x 5 cms and subsequently covered in both sides with a paper-based channel of the same size than the sensors. Then, an additional layer of adhesive plastic masks is surrounding both sides to seal the sensor and avoid any leaking and/or fluid entry/scape. Interestingly, a one-side sampling window located at the centre of the sensor is made with a known surface area (for example a circular 0.2 cm$^2$) to simply but also accurately captures the biological fluid present. To avoid any direct contact of the mammal skin and/or other vulnerable anatomical place a thin permeable membrane -as for example polycarbonate-covers the sampling window. Also, a minimum of two additional incisions along the longitudinal axis is presented to allocate the metal pads that are glued using any conductive glue available in the market. Finally, integration into a disposable wearable sweat patch is achieved allowing the signal processing to the electronic module as referenced in figures 13,14 and 15 of the present document, respectively.

Applications of the sensor

A second aspect of the present invention refers to a non-therapeutic method of detecting biological fluids, which comprises the following steps:

i) providing the sensor of the present invention; and

ii) contacting the sensor with a biological fluid.

The non-therapeutic method optionally comprises a further step of placing the sensor in contact with the skin of a human, mammal animals (such as horses) prior to contacting the sensor with biological fluids. Alternatively, the sensor can be in contact with an absorbent material -such as cotton, paper, etc.- that is in contact with the skin.
The biological fluid detected by the sensor includes fluids such as sweat, urine, blood and saliva, or any other liquid secretion from the body.
The biological fluids detected by a sensor of the present invention may be produced by a mammal such as a human, a horse, or any other mammal. In a preferred embodiment the mammal is a human.
The sensor may be disposable or non-disposable. The sensor may be used multiple times. Preferably the sensor is used once before being discarded.
The sensor is preferably in the form of a wearable device. Non-limiting examples of wearable devices include plasters, bandages, patches, diapers, clothing or footwear.
In one preferred embodiment of the second aspect of the present invention, the sensor of the invention is included in a modular sweat wearable platform to create a wide range of products across different markets. In this sense, sports, fitness, well-being, healthcare, food and beverages and the military sector are potential fields to monitor exercise/activity. Opportunities for the groups at higher risk of dehydration such as children, elderly, pregnant women, breastfeeding women, athletes or outdoor workers may be present. Also people that are undergoing some health problems, such as diarrhea or fever, may be prone to dehydration and thus prone to use the sensor to monitored sweat loss. In this sense, for children and elderly these sensors could help to educate of the importance of being hydrated. Cognitive impairment, bad mood, obesity, lower academic outcomes, hospitalizations or even mortality are some of the consequences of dehydration for these groups. For pregnant women hydration is crucial to support the growth of the fetus since water regulation dynamics are increased. In the case of breastfeeding women, hydration becomes particularly important since the production of breast milk significantly increases a mother's water loss. For athletes the sensors of the present invention could be used as tools suitable to monitor hydration and fluid intake during trainings in order to avoid the decrease of sports' performance and health related disorders such as heat stress or injuries. For health organizations and key industry players to have access to large sets of data on hydration status could represent a change in the current knowledge gap and facilitate supporting initiatives for improving the hydration of the population or personalized drinks.

Other field of interest are the cosmetic and beauty (deodorants, shampoos, perfumes, etc.) field and the textile sector (smart clothing, sport textiles, footwear, underwear, etc.), where this technology could be widely applied for different purposes. In this sense, a vast majority of the cosmetic and beauty products are applied regionally and/or to anatomical sensitive regions. For that reason and taking into account the realistic possibilities of our current technology this technology can provide solutions to skin care, skin hydration (moisture), skin perspiration (Onset of sweat, sweat amount, sweat patterns, sweat compositions), product development and effectiveness (benchmarking), marketing for big corporations and players, generation of novel indicators related with individual perspiration in order to increase and segment product's categories and therefore increase potential consumption.

In the medical field, the invention is targeted as a tool to overcome the treatment of hyperhidrosis, cystic fibrosis or to complement the detection of other diseases (i.e., monitoring the evolution or onset of health related events such as nightime hypoglicemia in diabetic patients). Interestingly this technology could be used to detect blood events or injuries or to help in the monitoring of wound healings. Furthermore, it could be used as a smart diaper (mainly for childcare and eldercare) to detect and monitor when a urine event takes place or to know when the diaper is full.

The following examples illustrate the present invention but do not limit the same.

### EXAMPLES

### Example 1. The sensor

Currently two modules may make up the sensor/s of the present invention. The first module of the sensor may be totally disposable and may incorporate a paper-based sensor (Figure 13 and Figure 14). This sensing strip shown in this example was prepared with the same procedures described for Figure 1. The second part of the sensor may be reusable and contained basically the connector holder and the electronic system that allows the signal processing and communication (Figure 15).

The parts and modules of the sensor can be easily simplified or enhanced depending on the application. Different embedded designs have been also proposed for diapers. The plug and play system allowing the connection between the disposable biosensor and the reusable module also forms part of the present invention.

### Example 2. Calculating the relative change of the electrical resistance ($\Delta R_{rel}$) of the sensors of the present inventon

To calculate the relative change of the electrical resistance of the biosensors, the first step is to make electrically conductive the absorbent commodity material used. Essentially, conductive ink (such as conductive ink made out of carbon nanotubes (CNT), Arabic gum, Indian ink and any suitable surfactants) is applied onto a suitable substrate, such as filter paper or any other absorbent-based material (cellulose), through a direct printing process. Once the ink has dried, the paper/absorbent material -which remains soft and flexible- becomes electrically conductive. This allows that, under suitable conditions, the biological fluid (sweat, urine, saliva, blood, tears) content affects the electrical resistance of this paper or absorbent-based material (Figure 12). The changes observed are rapid, and the phenomena are independent of any electrolyte concentration present in the fluid. Therefore, it is possible to correlate the measured electrical resistance with the loading of biological fluid as a function of the time. (Figure 3). For practical purposes, the relative change of the electrical resistance is used. The corresponding calibration plots, the relative change of resistance ($\Delta R\%$) as a function of liquid content ($\mu l$) can be used.

$$\Delta R_{rel}\% = \frac{(R_f - R_0)}{R_0} * 100$$

Therefore a standardized protocol of calibration with artificial sweat or other relevant biological fluid can be used with any given additions to ensure enough data points that cover the physiological range of interest As a result, a calibration equation is obtained and estimation of the quantity of fluid can be calculated.

## Example 3.

### 1.1. Short, medium and long-term manufacturing reproducibility analysis of the biosensors.

This experiment is designed to assess the reproducibility over time of the response obtained from a batch of sensors manufactured simultaneously after new conductive ink has been elaborated. The ink was composed by single-walled carbon nanotubes (SWCNT) dispersed in purified water (DDH$_2$O), single anionic surfactant sodium dodecyl benzenesulfonate (SDBS) and Arabic gum. Experimental tests were carried out immediately after 0, +1, +2, +3, +4, +6, +8 weeks in triplicate with the same experimental protocols and conditions. In that sense, a standardized calibration with artificial sweat from 0 up to 30 µl was performed with the following additions (1, 2, 2, 5, 5, 5, 10 µl respectively) upon resistance stabilization was achieved. This is important in order to observe whether or not sensors can be modified over time and therefore affect the response. The present results show a stable sensitivity over time even though a slight decrease of sensitivity can be seen in the calibration slopes of the graph (Figure 10). Implications for -calibration-free, scalability or shelf life may be taken into consideration.

### 1.2 Sweat-rate analysis of the biosensors.

This preliminary experiment was carried out to assess whether or not modifying the sweat rate and trying to simulate the skin flux the analytical parameters of the sensor could be affected. In this case, four constant sweat-rate (SR) based calibrations were performed. SR were selected based on low (0.14 µl·min$^{-1}$), medium (0.28 µl·min$^{-1}$, 0.43 µl·min$^{-1}$) and high (0.57 µl·min$^{-1}$) physiological perspiration rates (considering the area of the sensor). Additions every minute were accordingly conducted per each sweat-rate analysed. As we can see in figure 11 the difference in sweat amount is sensitive during the 35 min of the calibrations indicating that the sensor presents an excellent sensitivity despite of the SR. Furthermore the sensors present an excellent linear range in all the time points during the SR calibrations

### Example 4. Comparison of conductive inks.

This experiment was designed to compare the response of the sensor at low moisture levels (below 1.5 µL) using SWCNT ink alone and in combination also with Arabic gum and Indian ink. A total of 6 biosensors (3 SWCNT and 3 Hybrid biosensors) of 5 cms of length for 1 cm width were evaluated during the experiment. The protocol of the calibration consisted on artificial sweat additions of 0.15 µL·min$^{-1}$ up to a total of 1.2 µL. Results indicate that Hybrid ink present an excellent sensitivity with better limits of detection of moisture when compared to SWCNT ink alone. The results are shown in figure 16.

### Example 5:

This example illustrates the effect of using different types of carbon-based materials on the sensor response. The sensors used in these experiments table were prepared following the same procedures as described above, namely, a conductive substrate was made by painting a conventional filter paper (6 cm x 1 cm) with with conductive ink several times. The ink composition was changed as described in table 1. Once the conductive paper is dried, either water or artificial sweat are added and the resistance change is monitored as a function of the time. Triplicate analysis was carried out by each condition. Thereafter, the relative change in resistance obtained in each case is plotted against the volume of liquid added and the slope of this line is used to calculate the sensitivity of the sensor.

*Table 1*

| Entry | Carbon nanotube type | Quantity of Carbon nanotubes used (mg/ml) | Sensitivity (%/µL) |
|-------|---------------------|-------------------------------------------|--------------------|
| 1 | SWCNT | 3 | 1.4 ± 0.2 |
| 2 | MWCNT | 3 | 3.1 ± 0.5 |

(continued)

| Entry | Carbon nanotube type | Quantity of Carbon nanotubes used (mg/ml) | Sensitivity (%/μL) |
|---|---|---|---|
| 3 | SWCNT<br>Graphite nanofibers | 3<br>5 | 2.3 ± 0.1 |
| 4 | SWCNT<br>Graphite nanofibers | 3<br>10 | 2.4 ± 0.1 |
| 5 | SWCNT<br>Graphite nanofibers | 0.5<br>5 | 3.0 ± 0.1 |
| 6 | Graphite nanofibers | 3 | 9.2 ± 1.3 |

These results show that the nature of the carbon-based material used to make the ink has an influence on the sensitivity obtained. Multiwall carbon nanotubes display more sensitivity that single wall carbon nanotubes. Also, even higher sensitivity is obtained when graphite is used to make the ink. Nevertheless, making an homogeneous only-graphite ink presents several challeges. First, more layers are required to achieve a conductive material. Second, the drying of the ink is not homogeneous, which affects the reproducibility of the sensors (see standard deviation from ink number 6). This can be due to the lower interaction of graphite with the cellulose paper. For this reason, combination of graphite and SWCNT shows overall better results, even if the sensitivity is lower.

**Claims**

1. A non-therapeutic use of a sensor comprising a substrate coated with conductive ink, the substrate being inert relative to the conductive ink, the sensor comprising means for measuring conductivity or resistivity of the conductive ink, wherein the conductive ink comprises:

   i) a carbon substrate selected from the group comprising carbon nanostructure materials, amorphous carbon, graphite and combinations thereof;
   ii) a surfactant selected from the group comprising cationic surfactants, anionic surfactants and neutral surfactants; and
   iii) a polymer;

   for detecting biological fluids *in vitro.*

2. The non-therapeutic use according to claim 1, wherein the sensor is disposable.

3. The non-therapeutic use according to claims 1 or 2 wherein the polymer is a gum selected from the group comprising natural gum, Gum Arabic, chewing gum, and combinations thereof.

4. The non-therapeutic use according to any one of claims 1 to 3, wherein the carbon nanostructure materials are selected from carbon nanotubes.

5. The non-therapeutic use according to any one of claims 1 to 4, further comprising Indian ink.

6. The non-therapeutic use according to any one of claims 1 to 5, wherein the biological fluid is selected from the group consisting of sweat, urine blood and saliva.

7. The non-therapeutic use according to any one of claims 1 to 6, wherein the substrate is selected from the group consisting of paper, cotton, gum, carbon fibres, and combinations thereof.

8. The non-therapeutic use according to any one of claims 1 to 7, wherein the substrate is in the form of a wearable device such as a plaster, a bandage, a patch, a diaper, clothing or footwear.

9. The non-therapeutic use according to any one of claims 1 to 8, wherein the polymer is Gum Arabic.

10. The non-therapeutic use according to any one of claims 1 to 9, wherein the carbon substrate is graphite and no polymer is present in the conductive ink.

11. A non-therapeutic method of detecting biological fluids *in vitro* comprising the steps of:

i) providing a sensor as defined in any of claims 1 to 10; and
ii) contacting the sensor with a biological fluid *in vitro* selected from the group consisting of sweat, urine and saliva.

12. A sensor for detecting biological fluid comprising a substrate coated with conductive ink, the substrate being inert relative to the conductive ink, the sensor comprising means for measuring conductivity or resistivity of the conductive ink, wherein the conductive ink comprises:

i) graphite; and
ii) a surfactant selected from the group consisting of cationic surfactants, anionic surfactants and neutral surfactants.

13. The sensor for detecting biological fluid according to claim 12 further comprising a polymer selected from the group comprising polysacharids and gums.

14. The sensor for detecting biological fluid according to claim 13, where the polymer is a gum selected from the group consisting of natural gum, Gum Arabic, chewing gum, and mixtures thereof.

15. A sensor for use in treating wounds comprising a substrate coated with conductive ink, the substrate being inert relative to the conductive ink, the sensor comprising means for measuring conductivity or resistivity of the conductive ink, wherein the conductive ink comprises:

a) a carbon substrate selected from the group comprising carbon nanostructure materials, amorphous carbon, graphite and combinations thereof;
b) a surfactant selected from the group comprising cationic surfactants, anionic surfactants and neutral surfactants; and
c) a polymer;

wherein when the carbon substrate is graphite, optionally no polymer is present in the conductive ink film.

Fig. 1

Fig. 2

Fig. 3

Fig 4.

Fig. 5.

Sensor A - Resistance ($\Omega$) vs Sensor B - Resistance ($\Omega$)

$y = 1.0008x$
$R^2 = 0.9881$

Fig. 6

Sensor ($\mu$l) vs Cotton Patch ($\mu$l)

$y = 0.8516x - 0.8192$

Fig. 7

Fig. 8

Legend:
- ◆ 0.1%
- ▦ 1%
- ▲ 1.5%
- ✕ 2%
- ✻ 0.5%
- ● 0%
- Polinómica (0.1%)
- Polinómica (1%)
- Polinómica (1.5%)
- Polinómica (2%)
- Polinómica (0.5%)
- Polinómica (0%)

Fig. 9

Fig. 10.

Fig. 11

Fig. 12

Area < $0{,}2cm^2$

Fig 13

## MODULE 1. DISPOSABLE SENSOR

Transparent Film

Connector receptor

**Back View**

**Lateral View**

Absorbant

Sampling windows

Transparent Adhesive

**Front View**

Fig. 14

## DISPOSABLE SENSOR PARTS

Plastic Mask

Polycarbonate Membrane

Paper-based microfluidic channel

Polycarbonate Membrane

Paper-based microfluidic channel

Connector receptor

Absorbant

Transparent Adhesive

Fig. 15

## MODULE 2. REUSABLE SENSOR PART

Plastic holder

Electronic system

Conductive Super Magnets

**Lateral View**

Conductive Super Magnets

Plastic holder

**Back View**

Fig. 16

$y = 4.8939x + 1.3006$
$R^2 = 0.9947$

$y = 1.052x + 0.2294$
$R^2 = 0.8807$

Legend:
- ◆ SWCNT
- ▦ Hybrid Sensor (Arabic Gum + Indian Ink
- —— Linear (SWCNT)
- —— Linear (Hybrid Sensor (Arabic Gum + Indian Ink)

Axis labels: $\Delta$Rrel(%) (vertical), $\Delta$Sweat ($\mu$L) (horizontal)

Europäisches
Patentamt

European
Patent Office

Office européen
des brevets

**PARTIAL EUROPEAN SEARCH REPORT**

under Rule 62a and/or 63 of the European Patent Convention.
This report shall be considered, for the purposes of
subsequent proceedings, as the European search report

Application Number

EP 17 38 2045

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 999 786 B1 (HYPOGUARD LTD [GB]) 27 November 2002 (2002-11-27) * paragraphs [0032] - [0034], [0059] - [0075]; figures 1,30 * ----- | 1-14 | INV. A61B5/00 A61B5/02 A61B5/20 ADD. C09D11/52 |
| X | GUO Y ET AL: "Screen-printable surfactant-induced sol-gel graphite composites for electrochemical sensors", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER BV, NL, vol. 46, no. 3, 15 May 1998 (1998-05-15), pages 213-219, XP004147300, ISSN: 0925-4005, DOI: 10.1016/S0925-4005(98)00115-4 * abstract * * sections 2.2, 2.3.1, 2.3.2 and 3.1-3.5 * ----- | 1-14 | |
| X | US 2007/035405 A1 (WADA ICHIRO [JP] ET AL) 15 February 2007 (2007-02-15) * paragraphs [0038], [0039]; figures 1,8 * ----- -/-- | 1-14 | TECHNICAL FIELDS SEARCHED (IPC) A61B C09D |

## INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| The Hague | 30 October 2017 | Mecking, Nikolai |

EPO FORM 1503 03.82 (P04E07)

page 1 of 2

Europäisches
Patentamt

European
Patent Office

Office européen
des brevets

**PARTIAL EUROPEAN SEARCH REPORT**

Application Number

EP 17 38 2045

| | | DOCUMENTS CONSIDERED TO BE RELEVANT | | CLASSIFICATION OF THE APPLICATION (IPC) |
|---|---|---|---|---|
| Category | Citation of document with indication, where appropriate, of relevant passages | | Relevant to claim | |
| A | US 2012/172496 A1 (HAO HAI-YAN [CN] ET AL) 5 July 2012 (2012-07-05) * paragraphs [0012] - [0022]; figure 2 * ----- | | 1-14 | |
| A | The Dow Chemical Company: "TERGITOL NP-10 Surfactant Product Information", , 1 January 2009 (2009-01-01), XP55418488, Retrieved from the Internet: URL:http://www.dow.com/assets/attachments/ business/pcm/tergitol/tergitol_np-10/tds/n p-10.pdf [retrieved on 2017-10-24] * the whole document * ----- | | 1-14 | |

TECHNICAL FIELDS
SEARCHED          (IPC)

EPO FORM 1503 03.82 (P04C10)

3

Europäisches
Patentamt

European
Patent Office

Office européen
des brevets

**INCOMPLETE SEARCH
SHEET C**

Application Number

EP 17 38 2045

Claim(s) completely searchable:
        1-14

Claim(s) not searched:
        15

Reason for the limitation of the search:

In the present application the search division has identified multiple
independent claims in the same category, namely independent apparatus
claims 12 and 15. Therefore, in accordance with Rule 62a(1) EPC, the
applicant was invited to indicate the claims complying with Rule 43(2)
EPC on the basis of which the search is to be carried out.
In response to said invitation the applicant requested for the search
report to be drawn up, with respect to the independent claims pertaining
to the apparatus category, on the basis of independent claim 12.
The search was therefore carried out on the basis of claims 1-14.

## ANNEX TO THE EUROPEAN SEARCH REPORT
## ON EUROPEAN PATENT APPLICATION NO.

EP 17 38 2045

This annex lists the patent family members relating to the patent documents cited in the above-mentioned European search report.
The members are as contained in the European Patent Office EDP file on
The European Patent Office is in no way liable for these particulars which are merely given for the purpose of information.

30-10-2017

| Patent document cited in search report | | Publication date | Patent family member(s) | | | Publication date |
|---|---|---|---|---|---|---|
| EP 0999786 | B1 | 27-11-2002 | AT | 228334 | T | 15-12-2002 |
| | | | AU | 8547098 | A | 22-02-1999 |
| | | | DE | 69809752 | D1 | 09-01-2003 |
| | | | DE | 69809752 | T2 | 28-05-2003 |
| | | | EP | 0999786 | A1 | 17-05-2000 |
| | | | JP | 2001511384 | A | 14-08-2001 |
| | | | WO | 9905966 | A1 | 11-02-1999 |
| US 2007035405 | A1 | 15-02-2007 | EP | 1905400 | A1 | 02-04-2008 |
| | | | EP | 2478877 | A2 | 25-07-2012 |
| | | | JP | 4979999 | B2 | 18-07-2012 |
| | | | JP | 2007044493 | A | 22-02-2007 |
| | | | KR | 20080030021 | A | 03-04-2008 |
| | | | TW | I307275 | B | 11-03-2009 |
| | | | US | 2007035405 | A1 | 15-02-2007 |
| | | | WO | 2007007844 | A1 | 18-01-2007 |
| US 2012172496 | A1 | 05-07-2012 | CN | 102093774 | A | 15-06-2011 |
| | | | US | 2012172496 | A1 | 05-07-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140182372 A1

**Non-patent literature cited in the description**

- **MÄDER et al.** *J. Mater. Chem. A,* 2014, vol. 2, 5541-55447

- **XING et al.** *ACS Appl. Mater. Interfaces,* 2015, vol. 7, 26195-26205